(19)

Europäisches
Patentamt
European
Patent Office
Office européen
des brevets

(11) **EP 2 872 651 B1**

(12) **EUROPEAN PATENT SPECIFICATION**

(45) Date of publication and mention
of the grant of the patent:
**25.04.2018 Bulletin 2018/17**

(51) Int Cl.:
***C12Q 1/68*** (2018.01)

(21) Application number: **13752666.1**

(86) International application number:
**PCT/IB2013/001508**

(22) Date of filing: **11.07.2013**

(87) International publication number:
**WO 2014/009798 (16.01.2014 Gazette 2014/03)**

(54) **GENE EXPRESSION PROFILING USING 5 GENES TO PREDICT PROGNOSIS IN BREAST CANCER**

GENEXPRESSIONSPROFILIERUNG MIT 5 GENEN FÜR VORHERSAGE/PROGNOSEN BEI BRUSTKREBS

PROFILAGE D'EXPRESSION GÉNIQUE À L'AIDE DE 5 GÈNES POUR PRÉDIRE LE PRONOSTIC DANS LE CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.07.2012 PCT/IB2012/053582**

(43) Date of publication of application:
**20.05.2015 Bulletin 2015/21**

(73) Proprietor: **Alphagenics Biotech S.r.l.
16153 Genova (IT)**

(72) Inventors:
• **MORANDI, Luca**
**40139 Bologna (IT)**
• **SORMANI, Maria Pia**
**16132 Genova (IT)**
• **CENTELEGHE, Jean-Luc**
**1095 Lutry (CH)**

(56) References cited:
• **LAURA J VAN 'T VEER ET AL: "Gene Expression Profiling Predicts Clinical Outcome of Breast Cancer", NATURE: INTERNATIONAL WEEKLY JOURNAL OF SCIENCE (AND SUPPLEMENTARY INFORMATION), NATURE PUBLISHING GROUP, UNITED KINGDOM, vol. 415, no. 6871, 31 January 2002 (2002-01-31), pages 530-536, XP008138701, ISSN: 0028-0836, DOI: 10.1038/415530A cited in the application**

• **PAWITAN YUDI ET AL: "Gene expression profiling spares early breast cancer patients from adjuvant therapy: derived and validated in two population-based cohorts", BREAST CANCER RESEARCH, CURRENT SCIENCE, LONDON, GB, vol. 7, no. 6, 3 October 2005 (2005-10-03) , pages R953-R964, XP021011896, ISSN: 1465-5411, DOI: 10.1186/BCR1325 -& Y Pawitan ET AL: "Gene expression profiling spares early breast cancer patients from adjuvant therapy: derived and validated in two population-based cohorts. Supplementary Report", Breast Cancer Research, 3 October 2005 (2005-10-03), pages 1-12, XP055090275, Retrieved from the Internet: URL:http://breast-cancer-research.com/cont ent/7/6/R953/additional [retrieved on 2013-11-26]**

• **PAIK S ET AL: "A MULTIGENE ASSAY TO PREDICT RECURRENCE OF TAMOXIFEN-TREATED, NODE-NEGATIVE BREAST CANCER", NEW ENGLAND JOURNAL OF MEDICINE, MASSACHUSETTS MEDICAL SOCIETY, BOSTON, MA, US, vol. 351, no. 27, 30 December 2004 (2004-12-30), pages 2817-2826, XP008043033, ISSN: 1533-4406, DOI: 10.1056/NEJMOA041588**

• **LIAN-FANG LI ET AL: "Integrated gene expression profile predicts prognosis of breast cancer patients", BREAST CANCER RESEARCH AND TREATMENT, KLUWER ACADEMIC PUBLISHERS, BO, vol. 113, no. 2, 16 February 2008 (2008-02-16), pages 231-237, XP019671303, ISSN: 1573-7217, DOI: 10.1007/S10549-008-9925-4**

**(Cont. next page)**

- **GIORGIO MUSTACCHI ET AL: "Identification and Validation of a New Set of Five Genes for Prediction of Risk in Early Breast Cancer", INTERNATIONAL JOURNAL OF MOLECULAR SCIENCES, vol. 14, no. 5, 6 May 2013 (2013-05-06), pages 9686-9702, XP055090028, ISSN: 1661-6596, DOI: 10.3390/ijms14059686**

SEQUENCE LISTING

<110> QUALITY SYSTEMS MANAGEMENT & HEALTH S.r.l. (QSM S.r.l.)

<120> GENE EXPRESSION PROFILING USING 5 GENES TO PREDICT PROGNOSIS IN BREAST CANCER

<130> FT/PV/13507

<160> 44

<170> PatentIn version 3.5

<210> 1
<211> 21
<212> DNA
<213> artificial sequence

<220>
<223> oligo primer for qRT-PCR

<400> 1
atgagtatgc ctgccgtgtg a          21

<210> 2
<211> 20
<212> DNA
<213> artificial sequence

<220>
<223> oligo Primer reverse qRT-PCR

<400> 2
ggcatcttca aacctccatg         20

<210> 3
<211> 22
<212> DNA
<213> artificial sequence

<220>
<223> oligo primer forward qRT-PCR

<400> 3
ttgccgacag gatgcagaag ga        22

<210> 4
<211> 22
<212> DNA
<213> artificial sequence

<220>
<223> oligo primer reverse qRT-PCR

<400> 4
aggtggacag cgaggccagg at        22

<210> 5
<211> 21
<212> DNA
<213> artificial sequence

<220>
<223> oligo primer forward qRT-PCR

<400> 5
gaggacatct tccacgagca c          21

<210> 6
<211> 20
<212> DNA
<213> artificial sequence

<220>
<223> oligo primer reverse qRT-PCR

<400> 6
aggtagatgc ggcggtaggt          20

<210> 7
<211> 22
<212> DNA
<213> artificial sequence

<220>
<223> oligo primer forward qRT-PCR

<400> 7
ggtagtcaag tccggatcaa gg          22

<210> 8
<211> 22
<212> DNA
<213> artificial sequence

<220>
<223> oligo primer reverse qRT-PCR

<400> 8
tccagaacct tctcgatgaa ca          22

<210> 9
<211> 20
<212> DNA
<213> artificial sequence

<220>
<223> oligo primer forward qRT-PCR

<400> 9
agtacctgaa ccggcacctg          20

<210> 10
<211> 22
<212> DNA
<213> artificial sequence

<220>
<223> oligo primer reverse qRT-PCR

<400> 10
cagagacagc caggagaaat ca         22


<210> 11
<211> 22
<212> DNA
<213> artificial sequence


<220>
<223> oligo primer forward qRT-PCR


<400> 11
atgaagtaac tggctgggtg ct         22


<210> 12
<211> 20
<212> DNA
<213> artificial sequence


<220>
<223> oligo primer reverse qRT-PCR


<400> 12
tgaagcctgt ccttgggaat         20


<210> 13
<211> 22
<212> DNA
<213> artificial sequence


<220>
<223> oligo primer forward qRT-PCR


<400> 13
agcccttctc catggaaaca ta         22


<210> 14
<211> 22
<212> DNA
<213> artificial sequence


<220>
<223> oligo primer reverseqRT-PCR


<400> 14
cagctgactt ggaaggagag gt         22


<210> 15
<211> 20
<212> DNA
<213> artificial sequence


<220>
<223> oligo primer forward qRt-PCR


<400> 15
gttgccctgt ctgtcgtctg         20

<210> 16
<211> 20
<212> DNA
<213> artificial sequence

<220>
<223> oligo reverse qRT-PCR

<400> 16
cttgaggatg caggacaggt        20

<210> 17
<211> 19
<212> DNA
<213> artificial sequence

<220>
<223> oligo primer forward qRT-PCR

<400> 17
tgaagtgccc cttggacag        19

<210> 18
<211> 23
<212> DNA
<213> artificial sequence

<220>
<223> oligo primer reverse qRT-PCR

<400> 18
caggcccagt aaacactcaa aag        23

<210> 19
<211> 22
<212> DNA
<213> artificial sequence

<220>
<223> oligo primer forward qRT-PCR

<400> 19
ccctcaaaga gagaaacctg ga        22

<210> 20
<211> 22
<212> DNA
<213> artificial sequence

<220>
<223> oligo primer reverse qRT-PCR

<400> 20
atcaacaggc aacacaggat ct        22

<210> 21
<211> 23
<212> DNA

<213> artificial sequence

<220>
<223> oligo primer forward qRT-PCR

<400> 21
cgtgttctct ctggaaactg ttc        23

<210> 22
<211> 23
<212> DNA
<213> artificial sequence

<220>
<223> oligo primer reverse qRT-PCR

<400> 22
gaacgtacct cctcattgtc tgc        23

<210> 23
<211> 22
<212> DNA
<213> artificial sequence

<220>
<223> oligo primer forward qRT-PCR

<400> 23
ggaaaattca cacagcaaga ca        22

<210> 24
<211> 21
<212> DNA
<213> artificial sequence

<220>
<223> oligo primer reverseqRT-PCR

<400> 24
agaggccgta caagaagtgg t        21

<210> 25
<211> 20
<212> DNA
<213> artificial sequence

<220>
<223> oligo primer forward qRT-PCR

<400> 25
aacttctgct caggcccttg        20

<210> 26
<211> 20
<212> DNA
<213> artificial sequence

<220>

<223> oligo primer reverse qRT-PCR

<400> 26
aggcagatgc ttcagggttc          20

<210> 27
<211> 20
<212> DNA
<213> artificial sequence

<220>
<223> oligo primer forward qRT-PCR

<400> 27
ccgtgtctcg acttcctcct          20

<210> 28
<211> 20
<212> DNA
<213> artificial sequence

<220>
<223> oligo primer reverse qRT-PCR

<400> 28
cgttgagctc caggttctcc          20

<210> 29
<211> 21
<212> DNA
<213> artificial sequence

<220>
<223> oligo primer forward qRT-PCR

<400> 29
gattctacgc ctgcatccac t          21

<210> 30
<211> 22
<212> DNA
<213> artificial sequence

<220>
<223> oligo primer reverse qRT-PCR

<400> 30
ccctgctaag ttgtggtgtg aa          22

<210> 31
<211> 19
<212> DNA
<213> artificial sequence

<220>
<223> oligo primer forward qRT-PCR

<400> 31

gcaagctgga ctcggtctt          19

<210> 32
<211> 20
<212> DNA
<213> artificial sequence

<220>
<223> oligo primer reverse qRT-PCR

<400> 32
cctgtgtaca cccacacctg          20

<210> 33
<211> 21
<212> DNA
<213> artificial sequence

<220>
<223> oligo primer forward qRT-PCR

<400> 33
gaatccaggc acctctacca c          21

<210> 34
<211> 22
<212> DNA
<213> artificial sequence

<220>
<223> oligo primer reverse qRT-PCR

<400> 34
agtccagatg tctacggcat gg          22

<210> 35
<211> 22
<212> DNA
<213> artificial sequence

<220>
<223> oligo primer forward qRT-PCR

<400> 35
cagtttccag aagcagccag ag          22

<210> 36
<211> 22
<212> DNA
<213> artificial sequence

<220>
<223> oligo primer reverse qRT-PCR

<400> 36
gaggatttgc tgaggtcatt ta          22

<210> 37

<211> 22
<212> DNA
<213> artificial sequence

<220>
<223> oligo primer forward qRT-PCR

<400> 37
cagtggtcac ctcacactcc tc          22

<210> 38
<211> 23
<212> DNA
<213> artificial sequence

<220>
<223> oligo primer reverse qRT-PCR

<400> 38
tttgtcattt gcttctattt cca          23

<210> 39
<211> 21
<212> DNA
<213> artificial sequence

<220>
<223> oligo primer forward qRT-PCR

<400> 39
tatcagcccc catctaccaa c          21

<210> 40
<211> 22
<212> DNA
<213> artificial sequence

<220>
<223> oligo primer reverse qRT-PCR

<400> 40
tcttgtttcc tgcactccct ct          22

<210> 41
<211> 23
<212> DNA
<213> artificial sequence

<220>
<223> oligo primer forward qRT-PCR

<400> 41
tcctcagaga gtagcagctc aca          23

<210> 42
<211> 21
<212> DNA
<213> artificial sequence

<220>
<223> oligo primer reverse qRT-PCR

<400> 42
ccttgatgat tcccaggagt t          21

<210> 43
<211> 22
<212> DNA
<213> artificial sequence

<220>
<223> oligo primer forward qRT-PCR

<400> 43
ccaggaaatt agcaagggaa ag          22

<210> 44
<211> 24
<212> DNA
<213> artificial sequence

<220>
<223> oligo primer reverse qRTpCR

<400> 44
cttgtctgca tagacttctt ctca          24

**Description**

**Technical field**

The present disclosure relates to methods, kits and systems for the prognosis of the disease outcome of breast cancer. More specific, the present invention relates to the prognosis of breast cancer based on measurements of the expression levels of marker genes in tumor samples of breast cancer patients. Thus, the invention concerns a quantitative RT-PCR method to measure a series of mRNA levels in biopsied breast tumor tissues, including archived paraffin-embedded biopsy material. The invention also allows to define three signature related to prognosis using an algorithm of choice: high risk/poor prognosis; medium risk/medium prognosis; low risk/good prognosis. Obtained gene expression profile will be important for assigning the most optimal treatment options to breast cancer patient based upon knowledge derived from gene expression studies.

**Background of the invention**

In the last few years, several multi-gene assays performed on tumor tissue from women with early breast cancer have been proposed to provide prognostic information and discriminate good vs. poor prognosis . These assays might be useful to assist in making more informed treatment decisions regarding chemotherapy, according to the main international guidelines.

The array gene expression analysis "Mammaprint®" identifies a 70 gene-signature indicative for poor prognosis in patients with lymph node-negative disease or with 1-3 positive nodes, predicting chemotherapy benefit in the "high risk" group, vs. no apparent benefit in the "low risk" group, in a non-randomized clinical setting. It needs fresh/frozen tissue of the primary breast tumors. The multigene assay "Oncotype DX®" evaluate gene expression analysis of 21 genes starting from paraffin-embedded tissue calculating a recurrence score to classify patients at low, intermediate, or high risk for recurrence. From two independent retrospective analyses from phase III clinical trial with adjuvant tamoxifen-alone control arms, the 21-gene recurrent score (RS) assay defines a group of patients with low scores who do not appear to benefit from chemotherapy, and a second group with very high scores who derive major benefit from chemotherapy, independently of age and tumor size. Other studies using a supervised approach based on clinical outcome endpoint to tumor grade as a basis for gene findings have resulted in development of multiple commercial reference lab assays for prognostication (MapQuant Dx, Theros Breast Cancer Index).

The above-mentioned multigene assays are expensive and validations have been made on patients selected by age and nodal or Estrogen Receptor status and or received adjuvant treatment.

More recently, multigene assays have been shown to provide information superior or additional to the standard clinical risk factors. It is generally recognized, that proliferation markers seem to provide the dominant prognostic information. Prominent examples of those predictors are the above cited Mammaprint test from Agendia, the Relapse Score from Veridex and the Genomic Grade Index, developed at the institute Jules Bordet and licensed to Ipsogen. All of these assays are based on determination of the expression levels of at least 70 genes and all have been developed for RNA not heavily degraded by formalin fixation and paraffin embedding, but isolated from fresh tissue (shipped in RNALaterTM) . Another prominent multigene assay is the Recurrence Score test of Genomic Health Inc. The test determines the expression level of 16 cancer related genes and 5 reference genes after RNA extraction from formalin fixed and paraffin embedded tissue samples. However, the current tools suffer from a lack of clinical validity and utility in the most important clinical risk group, i.e. those breast cancer patients of intermediate risk of recurrence based on standard clinical parameter. Therefore, better tools are needed to optimize treatment decisions based on patient prognosis. For the clinical utility of avoiding chemotherapy, a test with a high sensitivity and high negative predictive value is needed, in order not to undertreat a patient that eventually develops a distant metastasis after surgery.

In regard to the continuing need for materials and methods useful in making clinical decisions on adjuvant therapy, the present invention fulfills the need for advanced methods for the prognosis of breast cancer on the basis of readily accessible clinical and experimental data.

**Brief description of figures**

Figure 1 shows the multistep approach of the invention for the identification of the five informative genes.
Figure 2 shows the curves associated with DFS according to the three risk groups.
The score was categorized in three groups according to the tertiles of its distribution.
Figure 3 shows the curves associated with DFS after validation set.
Figure 4 shows the Primer sequences, slope, PCR efficiency and RSq of each of the 20 genes + 2 housekeeping genes used for qRT-PCR.

## Description of the invention

The present invention provides a method to assess the risk of recurrence of breast cancer.

The present invention relates to a method of determining the prognosis of a patient suffering from breast cancer, comprising the step of measuring the level of expression of mRNAs of the *FGF18*, *BCL2*, *PRC1*, *MMP9,* and SERF1a genes in an isolated biological sample of said patient consisting of a tumour or a tumour biopsy, or neoplastic cells, or paraffin embedded tumour or formalin fixed tissue specimen (FFPE) containing tumour sample of said patient, wherein a high *BCL2 and SERF1a* expression is associated with a poor prognosis and a decreased likelihood of long-term survival without breast cancer recurrence, and high *FGF18, PRC1 and MMP9* expression is associated with a good prognosis and an increased likelihood of long-term survival without breast cancer recurrence following surgical removal of the primary tumour. The method of the invention further comprises (a) assaying an expression level of mRNA transcripts of the group consisting of: *FGF18, BCL2, PRC1, MMP9, SERF1a;* (b) determining normalized expression levels of the RNA transcripts, wherein the normalized expression level of the RNA transcripts correlates with the likelihood of breast cancer recurrence; and (c) providing information comprising the likelihood of long-term survival without breast cancer recurrence for the patient, wherein the information comprises the normalized expression level of the RNA transcript or its expression product.

The method disclosed herein for determining the prognosis of a patient suffering from breast cancer may comprise:

(a) determining in a tumor sample from said patient the RNA expression levels of at least 2 of the following 5 genes: *FGF18, BCL2, PRC1, MMP9, SERF1a*

(b) mathematically combining expression level values for the genes of the said set which values were determined in the tumor sample to yield a combined score, wherein said combined score is indicative of a prognosis of said patient.

In one embodiment at least 2, preferably 3, more preferably 4, or all 5 *FGF18, BCL2, PRC1, MMP9, SERF1a* genes are selected. Also disclosed herein is a kit for determining the prognosis of a patient suffering from breast cancer, by measuring the level of expression of *FGF18, BCL2, PRC1, MMP9, SERF1a,* in a biological sample of said patient, wherein a high *BCL2 and SERF1a* expression is associated with a poor prognosis and a decreased likelihood of long-term survival without breast cancer recurrence, and/or high *FGF18, PRC1 and MMP9* expression is associated with a good prognosis and an increased likelihood of long-term survival without breast cancer recurrence.

According to an aspect of the disclosure there is provided a method as described above, wherein a risk of developing recurrence is predicted.

According to an aspect of the disclosure there is provided a method as described above, wherein said expression level is associated with a Gene Signature Score which predicts the risk of developing recurrence of breast cancer within 5 years.

According to an aspect of the disclosure there is provided a method as described above, wherein said expression level is determined as an RNA expression level.

According to an aspect of the disclosure there is provided a method as described above, wherein said expression level is determined by a PCR based method, preferably quantitative RT-PCR.

According to an aspect of the disclosure there is provided a method as described above, wherein said determination of expression levels is carried out on different kinds of samples for example formalin-fixed paraffin embedded tumor sample , a fresh-frozen tumor sample, biopsy sample.

According to an aspect of the disclosure there is provided a method as described above, wherein the expression level of said at least on marker gene (i.e. *FGF18, BCL2, PRC1, MMP9, SERF1a*) is determined as a pattern of expression relative to at least one reference gene or to a computed average expression value.

According to an aspect of the disclosure there is provided a method as described above, wherein said step of mathematically combining comprises a step of applying an algorithm to values representative of an expression level of a given gene.

According to an aspect of the disclosure there is provided a method as described above, wherein said algorithm is a linear combination of said values representative of an expression level of a given gene. According to the invention the expression levels of *FGF18, BCL2, PRC1, MMP9*, *SERF1a* were combined in a weighted linear score

$$GeneSignature\ score = \sum_i w_i\ value_i$$

wherein $w_i$ is weights determined by the coefficients of the multivariate Cox regression model (B values showed in several tables of the present invention) and value$_i$ represents the gene expression levels. The tertile values of the signature were chosen as the cut offs to classify patients at low, medium or high risk of disease relapse. The correlation of the two signatures with DFS was evacuate by Kaplan Meier (KM) survival curves and by the Hazard estimate by a Cox model (Figures). The term "tertile" in the present description has the meaning of any of the two points that divide an ordered distribution into three parts, each containing a third of the population.

According to an aspect of the disclosure there is provided a method as described above, wherein a value for a representative of an expression level of a given gene is normalized against a reference set comprising RNA transcripts of at least two genes chosen among housekeeping genes *ACTB, GAPDH, B2M, TBP* and/or or their expression products by GeNorm or the statistical computing language *R*.

According to a preferred aspect the disclosure provides a method as described above wherein the Gene Signature Score is determined normalized the expression level of the RNA transcript of *FGF18, BCL2, PRC1, MMP9, SERF1a* or its expression product, wherein the normalized expression level of the RNA transcript or its expression product allows the calculation of the following algorithm:

$$\text{Gene Signature Score} = 0.125\text{x } FGF18 - 0.560 \text{ x} BCL2 + 0.409\text{x } PRC1 + 0.104\text{x }$$
$$MMP9 - 0.188 \text{ x SERF1a.}$$

Also disclosed is the Gene Signature Score obtainable by the method disclosed herein. Gene Signature Score may categorize 3 groups according to the tertiles of its distribution:

Low Risk of recurrence within 5 years: *GeneSignatureScore* <-0.87
Intermediate Risk of recurrence within 5 years: -0.87 < *GeneSignatureScore* < -0.13
High Risk of recurrence within 5 years: *GeneSignatureScore* >=-0.13

The Gene Signature Score is associated with the risk of breast cancer recurrence in patient. In detail, a Gene Signature Score value of about "< -0.87" indicates a low risk of recurrence within 5 years, a Gene Signature Score value comprises among -0.87 < *GeneSignatureScore* < -0.13 represents an intermediate risk of recurrence within 5 years and a Gene Signature Score value of about $\geq$ - 0.13 represents an high risk of recurrence within 5 years.

According to an aspect of the disclosure there is provided a method as described above, wherein an information regarding nodal status of the patient and/or therapy treatment of patient is not necessary to determine the Gene Signature Score and for the mathematically combining expression level values for the genes to yield a combined score.

Prognosis is a medical term to describe the likely outcome of an illness. Here, a good prognosis correlates low risk of recurrence of cancer within five years, medium prognosis correlated with intermediate risk of recurrence cancer within five years and poor prognosis correlates with high risk of recurrence cancer within five years.

The disclosure further relates to a kit for performing a method as described above, said kit comprising a set of oligonucleotides capable of specifically binding sequences or fragments of at least one gene *FGF18, BCL2, PRC1, MMP9,SERF1a* as well as at least one housekeeping genes above indicated.

The disclosure further relates to a computer program product capable of processing values representative of an expression level of a combination of genes mathematically combining said values to yield a combined score, wherein said combined score is indicative of risk of cancer recurrence in said patient, according to the above methods.

Said computer program product may be stored on a data carrier or implemented on a diagnostic system capable of outputting values representative of an expression level of a given gene, such as a real time PCR system.

If the computer program product is stored on a data carrier or running on a computer, operating personal can input the expression values obtained for the expression level of the respective genes. The computer program product can then apply an algorithm to produce a combined score indicative of risk of cancer recurrence for a given patient.

The methods of the present invention have the advantage of providing a reliable prediction of an outcome of disease based on the use of only a small number of genes. The methods of the present invention have been found to be especially suited for prognostic signature for disease free survival in breast cancer patients where the indication for adjuvant chemotherapy added to endocrine treatment is uncertain.

## Detailed description of the invention

Applicant has now surprisingly found that the method of the invention is able to predict the likelihood of long-term survival of a breast cancer patient without the recurrence of breast cancer, following surgical removal of the primary tumor, comprising analysis of the expression level of the following 5 mRNA including: *FGF18, BCL2, PRC1, MMP9,SERF1a,* wherein overexpression of *BCL2 and SERF1a,* indicates a decreased likelihood of longterm survival without breast cancer recurrence, and the overexpression of *FGF18, PRC1 and MMP9,* indicates an increased likelihood of long-term survival without breast cancer recurrence.

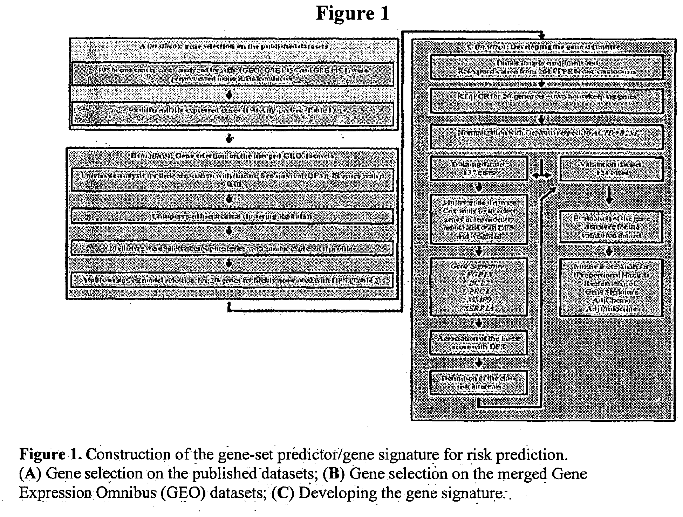

This method has a good discriminating ability and it is able to determine a prognostic signature for DFS (disease free survival) in breast cancer patients. The method of the invention allows the identification of five-gene recurrence score able to estimate the likelihood of recurrence in a series of consecutive breast cancer tissue samples. These five informative genes were selected by a multistep approach summarized in Figure 1. Firstly, we identified *in silico* a subset of 20 mRNA differentially regulated in breast cancer analyzing several publicly available array gene expression data using R/Bioconductor package. We further evaluated, *in vitro,* the expression level of these 20 genes in 261 consecutive invasive breast cancer cases not selected for age, adjuvant treatment, nodal and estrogen receptor status from paraffin embedded sections. The only requested feature was a minimum follow up of 5 years with full clinical data. Each tissue block was reviewed by a pathologist to ensure greater than 70% content of tumor cells. The gene expression analysis was based on quantitative RT-PCR. The biological samples dataset was split into a training and a validation dataset. The gene signature was developed on the training set by a multivariate stepwise Cox analysis selecting five genes independently associated with disease free survival (DFS). These five genes were combined into a linear score (signature) weighted according to the coefficients of the Cox model. The signature was then evaluated on the validation set assessing the discrimination ability by a Kaplan Meier analysis, using the same cut offs classifying patients at low, intermediate or high risk of disease relapse as defined on the training set. The five genes of the invention are unexpected selected from several genes. In fact the five genes of the invention are identified without any *a priori* selection for gene function or cancer involvement, but trough the correlation gene signature , between their expression level and DFS. The five genes of the invention able to correlate with a prognosis of recurrence of cancer are *FGF18, BCL2, PRC1, MMP9, SERF1a.* The function of *SERF1a* is still unknown, the other genes play an important role in cancer:

> (a) *FGF18* is over-expressed in tumors. *FGF18* expression is up-regulated through the constitutive activation of the *Wnt* pathway observed in most colorectal carcinomas. As a secreted protein, FGF18 can thus affect both the tumor and the connective tissue cells of the tumor microenvironment;
> (b) *BCL2:* Over-expression of BCL2 protein has been identified in a variety of solid organ malignancies, including breast cancer. *BCL2* transcript over-expression is related to unfavorable prognosis;
> (c) *PRC1*: It associates with the mitotic spindle and has been found to play a crucial role in the completion of cytokinesis. *PRC1* is negatively regulated by p53 and it is over-expressed in p53 defective cells suggesting that the gene is tightly regulated in a cancer-specific manner;
> (d) *MMP9:* Metalloproteases are frequently up-regulated in the tumor microenvironment. *MMP9* influence many aspects of tissue function by cleaving a diverse range of extracellular matrix, cell adhesion, and cell surface receptors, and regulate the bioavailability of many growth factors and chemokines;
> (e) *SERF1a:* The function of *SERF1a* is not already known. According to this disclosure there is a surprising and unexpected biological property of this gene in cancer. The method of the invention establish a link between our proposed molecular signature of breast cancer and the underlying capabilities acquired during the multistep development of human tumors.

The method of the invention has several advantages, for example it is easily used in the scientific and diagnostic laboratories, affordable, not time consuming, able to detect RNA in different samples e.g. it is able to detect the expression level of five genes of the invention in FFPE tissue and it might be performed easily in almost all laboratories with the required qRT-PCR instrumentations.

The method of the invention is validated on a "real life" clinical setting with a set of consecutive breast cancer cases irrespectively from age, nodal and estrogen receptor status, adjuvant treatment with at least a minimum follow up of 5 years.

The method of the invention is able to detect the expression level of five genes of the invention also in 74.6% of the initial set of cases due to RNA degradation from FFPE tissues according to the literature regarding other signatures RNA degradation can be monitored simply evaluating the Ct values of the housekeeping genes used for normalization.

According to further aspect of the disclosure the method above described is able to analyse the predictive value of the five-gene signature in ER positive population of tamoxifen alone benefit and of chemotherapy added to tamoxifen. The method should be useful proposed as non expensive prognostic signature for disease free survival in breast cancer patients where the indication for adjuvant chemotherapy added to endocrine treatment is uncertain. The present disclosure provides a prognostic tool for early breast cancer based on the analysis of the relative expression level of at least one *FGF18, BCL2, PRC1, MMP9* and *SERF1A* genes the gene signature score according to the invention has a good discriminating ability when tested on the validation set. The present disclosure thus relates to methods for determining the prognosis for survival, and for testing the effect of treatment regimens. The disclosure also relates to a kit for determining the prognosis for survival, and for testing the effect of treatment regimens.

Unless defined otherwise, technical and scientific terms used herein have the same meaning as commonly understood by one of ordinary skill in the art to which this invention belongs.

The inventors have now found that there is a correlation between the expression level of *FGF18, BCL2, PRC1, MMP9, SERF1a* genes and the prognosis for recurrence of cancer within five years.

At the best of our knowledge, no previous study has examined the relationship between the expression of these mRNAs and a patient's prognosis in cancer. This inventive idea was tested because we have been the first to observe that this genes were deregulated in cancer cell types. However, and importantly, these results were not obvious because many genes shown to be de-regulated in cancer have no predictive value toward a patient survival, recurrence or response to therapy. This is an important area of research in cancer because there are little data to help physicians chose which treatment is best for which patients or to determine the risk of recurrence of cancer.

The meanings of terms used in the present description are explained herein below.

The words "method" and "process" are interchangeable in the present description and both terms are used indifferently to refer and claim the present invention.

The term "node positive", "diagnosed as node positive", "node involvement" or "lymph node involvement" means a patient having previously been diagnosed with lymph node metastasis. It shall encompass both draining lymph node, near lymph node, and distant lymph node metastasis.

A "tumor sample" is a biological sample containing tumor cells, whether intact or degraded. The sample may be of any biological tissue or fluid. Such samples include, but are not limited to, tissue, core or fine needle biopsy samples, cell-containing body fluids, or cells isolated therefrom. This may also include sections of tissues such as frozen or fixed sections taken for histological purposes or microdissected cells or extracellular parts thereof.

A "gene" is a set of segments of nucleic acid that contains the information necessary to produce a functional RNA product. A "gene product" is a biological molecule produced through transcription or expression of a gene, e.g. an mRNA, cDNA or the translated protein.

An "mRNA" is the transcribed product of a gene and shall have the ordinary meaning understood by a person skilled in the art. A "molecule derived from an mRNA" is a molecule which is chemically or enzymatically obtained from an mRNA template, such as cDNA.

The term "expression level" refers to a determined level of gene expression. This may be a determined level of gene expression as an absolute value or compared to a reference gene (e.g. a housekeeping gene), to the average of two or more reference genes, or to a computed average expression value or to another informative gene without the use of a reference sample. The expression level of a gene may be measured directly, e.g. by obtaining a signal wherein the signal strength is correlated to the amount of mRNA transcripts of that gene or it may be obtained indirectly at a protein level.

The term "mathematically combining expression levels", within the meaning of the invention shall be understood as deriving a numeric value from a determined expression level of a gene and applying an algorithm to one or more of such numeric values to obtain a combined numerical value or combined score.

An "algorithm" is a process that performs some sequence of operations to produce information.

A "score" is a numeric value that was derived by mathematically combining expression levels using an algorithm. It may also be derived from expression levels and other information, e.g. clinical data. A score may be related to the outcome of a patient's disease.

A "discriminant function" is a function of a set of variables used to classify an object or event. A discriminant function thus allows classification of a patient, sample or event into a category or a plurality of categories according to data or parameters available from said patient, sample or event. Such classification is a standard instrument of statistical analysis well known to the skilled person. E.g. a patient may be classified as "high risk" or "low risk", "intermediate risk" according to data obtained from said sample.

The method of the invention is explained in the following paragraphs in more detailed manner.

The inventors, starting from the identification of a subset of 20 mRNA differentially regulated in breast cancer and analyzing several publicly available array gene expression data using R/Bioconductor package, have been isolated five genes able to predict the risk of cancer recurrence. Using q RT-PCR inventors evaluate 261 consecutive invasive breast cancer cases not selected for age, adjuvant treatment, nodal and estrogen receptor status from paraffin embedded sections. The biological samples dataset was split into a training (137 cases) and a validation set (124 cases). The gene signature was developed on the training set and a multivariate stepwise Cox analysis selected five genes independently associated with DFS: *FGF18* (HR = 1.13, $p$ = 0.05), *BCL2* (HR = 0.57, $p$ = 0.001), *PRC1* (HR = 1.51, $p$ = 0.001), *MMP9* (HR = 1.11, $p$ = 0.08), *SERF1a* (HR = 0.83, $p$ = 0.007).

These five genes were combined into a linear score (signature) weighted according to the coefficients of the Cox model, as: 0.125*FGF18* - *0.560BCL2* + 0.409*PRC1* + *0.104MMP9* - 0.188*SERF1A* (HR = 2.7, 95% CI = 1.9-4.0, $p$ < 0.001). The signature was then evaluated on the validation set assessing the discrimination ability by a Kaplan Meier analysis, using the same cut offs classifying patients at low, intermediate or high risk of disease relapse as defined on the training set ($p$ < 0.001). The gene signature score obtained according to the method of the invention is useful as prognostic signature for disease free survival in breast cancer patients where the indication for adjuvant chemotherapy added to endocrine treatment is uncertain.

**Genes to be included in the analysis**

To select the candidate genes to be included in the signature and to be analysed in biological samples by RealTime PCR, we used data deposited in NCBI Gene Expression Omnibus (GEO; available at http://www.ncbi.nlm.nih.gov/geo/, GEO Series accession number GSE1456 and GSE3494), including 408 breast cancer patients. Files containing raw intensity data of Affymetrix HU133A and HU133B arrays of the two (GSE1456 and GSE3494) were preprocessed using R/Bioconductor (GCRMA package, quantile normalization, median polish summarization). The two data sets were pre-processed together using the supercomputer Michelangelo (www.litbio.org). The candidate genes were selected from the above mentioned datasets as those included in 4 previously proposed signatures: the "70 gene good versus poor out come, A gene-expression signature as a predictor of survival in breast cancer." (Van de Vijver et al. and van't Veer J.L. et al., 2002, http://www.ncbi.nlm.nih.gov/pubmed/12490681) including 70 genes, the "recurrence-score" (Paik et al, 2004, http://www-ncbi.nlm.nih.gov/pubmed/15591335) including 21 genes, the "two-gcnc-ratio model" (Ma XJ, Wang et al, 2004, http://www.ncbi.nlm.nih.gov/pubmed/15193263) including 2 genes and the "Insuline resistence" (Kosntantopoulos Nicky at Al, 2011, https://www.researchgate.net/publication/47809153_A_gene_expression_signature_for_insuli n resistance) signature including 15 genes. Since some genes are present in more than one signature, the final extracted set was made up of 98 genes (194 Affyprobes) (Table 1).

Table 1: Genes selected

| Symbol | AffyID | Group | Affychip | Symbol | AffyID | Group | Affychip | Symbol | AffyID | Group | Affychip |
|--------|--------|-------|----------|--------|--------|-------|----------|--------|--------|-------|----------|
| ALDH4A1 | 203722_at | 1.00 | A | MKI67 | 212021_s_at | 2.00 | A | MCM6 | 201930_at | 1.00 | A |
| AP2B1 | 200612_s_at | 1.00 | A | MKI67 | 212022_s_at | 2.00 | A | MELK. | 204825_at | 1.00 | A |
| AP2B1 | 200615_s_at | 1.00 | A | MKI67 | 212023_s_at | 2.00 | A | MKI67 | 212020_s_at | 2.00 | A |
| AURKA | 204092_s_at | 2.00 | A | MMP11 | 203876_s_at | 2.00 | A | SLC2A3 | 240055_at | 1.00 | |
| AURKA | 208079_s_at | 2.00 | A | MMP11 | 203878_s_at | 2.00 | A | ZNF533 | 229019_at | 1.00 | |
| AURKA | 203080_at | 2.00 | A | MMP9 | 203936_s_at | 1.00 | A | ZNF533 | 243929_at | 1.00 | |
| AYTL2 | 201818_at | 1.00 | A | MYBL2 | 201710_at | 2.00 | A | IGF1 | 209540_at | 3.00 | A |
| BAG1 | 202387_at | 2.00 | A | NDC80 | 204162_at | 1.00 | A | IGFIR | 203628_at | 3.00 | A |
| BAG1 | 211475_s_at | 2.00 | A | NUSAPI | 218039_at | 1.00 | A | IGF2 | 202410_x_at | 1.00 | A |
| BBC3 | 211692_s_at | 1.00 | A | ORC6L | 219105_x_at | 1.00 | A | IGFBP4 | 201508_at | 3.00 | A |
| BC045642 | 212248_at | 1.00 | A | OXCT1 | 202780_at | 1.00 | A | IGFBP5 | 203424_s_at | 1.00 | A |
| BC045642 | 212250_at | 1.00 | A | PALM2-AKAP2 | 202759_s_at | 1.00 | A | IGFBP5 | 203425_s_at | 1.00 | A |
| BC045642 | 212251_at | 1.00 | A | PALM2-AKAP2 | 202760_a_at | 1.00 | A | IGFBP5 | 203426_s_at | 1.00 | A |
| BCL2 | 203684_s_at | 2.00 | A | PECI | 218025_s_at | 1.00 | A | IGFBP5 | 211958_at | 1.00 | A |
| BCL2 | 203685_at | 2.00 | A | FOR | 208305_at | 2.00 | A | IGFBP5 | 211959_at | 1.00 | A |
| BCL2 | 207004_at | 2.00 | A | PITRM1 | 205273_s_at | 1.00 | A | IGFBP6 | 203851_at | 3.00 | A |
| BCL2 | 207005_s_at | 2.00 | A | PQLC2 | 220453_at | 1.00 | A | IGFBP7 | 201163_s_at | 3.00 | A |
| BF034907 | 206023_at | 1.00 | A | PRC1 | 218009_s_at | 1.00 | A | IL17RB | 219255_x_at | 4.00 | A |
| BIRC5 | 202094_at | 2.00 | A | RAB6A | 201045_s_at | 1.00 | A | IL6ST | 204863_s_at | 3.00 | A |
| BIRC5 | 202095_s_at | 2.00 | A | RAB6A | 201047_x_at | 1.00 | A | INSIGI | 201627_s_at | 3.00 | A |
| BIRC5 | 210334_x_at | 2.00 | A | RAB6A | 201048_x_at | 1.00 | A | IRS1 | 204686_at | 3.00 | A |
| C16orf61 | 218447_at | 1.00 | A | RAB6A | 210406_s_at | 1.00 | A | IRS2 | 209184_s_at | 3.00 | A |
| C20orf46 | 219958_at | 1.00 | A | RFC4 | 204023_at | 1.00 | A | IGP2 | 219364_at | 1.00 | A |
| C9orf30 | 205112_at | 1.00 | A | SCUBE2 | 219197_s_at | 1.50 | A | LOC643008 | 229740_at | 1.00 | B |
| C9orf30 | 205123_s_at | 1.00 | A | SERFIA | 219982_s_at | 1.00 | A | MCM6 | 238977_at | 1.00 | B |
| CCNB1 | 214710_s_at | 2.00 | A | SIC2A3 | 202497_x_at | 1.00 | A | MS4A7 | 223343_at | 1.00 | B |
| CCNE2 | 205034_at | 1.00 | A | SLC2A3 | 202498_s_at | 1.00 | A | MS4A7 | 223344_s_at | 1.00 | B |
| CCNE2 | 211814_s_at | 1.00 | A | SIC2A3 | 202499_s_at | 1.00 | A | MS4A7 | 224358_s_at | 1.00 | B |
| CD68 | 203507_at | 2.00 | A | SLC2A3 | 216236_s_at | 1.00 | A | PALM2-AKAP2 | 226694_at | 1.00 | B |
| COC42BPA | 214464_at | 1.00 | A | SLC2A3 | 222088_s_at | 1.00 | A | QSOX2 | 227146_at | 1.00 | B |
| CENPA | 204962_s_at | 1.00 | A | STK32B | 219686_at | 1.00 | A | QSOX2 | 235239_at | 1.00 | B |
| CENPA | 210821_x_at | 1.00 | A | TGFB3 | 209747_at | 1.00 | A | RTN4RL1 | 229097_at | 1.00 | B |
| COL4A2 | 211964_at | 1.00 | A | TNFRSF10B | 209295_at | 3.00 | A | RTN4RL1 | 232596_at | 1.00 | B |

EP 2 872 651 B1

| Symbol | AffyID | Group | Affychip | Symbol | AffyID | Group | Affychip | Symbol | AffyID | Group | Affychip |
|---|---|---|---|---|---|---|---|---|---|---|---|
| COL4A2 | 211966_at | 1.00 | A | TNFRSF12A | 218368_s_at | 3.00 | A | RTN4RL1 | 242102_at | 1.00 | B |
| CTSL2 | 210074_at | 2.00 | A | TNFRSF21 | 214581_s_at | 3.00 | A | RUNDC1 | 226298_at | 1.00 | B |
| DCK | 203302_at | 1.00 | A | TNFSF10 | 214329_x_at | 3.00 | A | RUNDC1 | 235040_at | 1.00 | B |
| DIAPH3 | 220997_s_at | 1.00 | A | TSPYL5 | 213122_at | 1.00 | A | SERF1A | 223538_at | 1.00 | B |
| DTL | 218585_s_at | 1.00 | A | UCHL5 | 219960_s_at | 1.00 | A | SERF1A | 223539_s_at | 1.00 | B |
| ECT2 | 219787_s_at | 1.00 | A | WISP1 | 206796_at | 1.00 | A | SLC2A3 | 236180_at | 1.00 | B |
| EGLN1 | 221497_x_at | 1.00 | A | WISP1 | 211312_s_at | 1.00 | A | SLC2A3 | 236571_at | 1.00 | B |
| ESM1 | 208394_x_at | 1.00 | A | AA834945 | 230365_at | 1.00 | B | GRB7 | 210761_s_at | 2.00 | A |
| ESR1 | 205225_at | 2.00 | A | AA834945 | 235039_x_at | 1.00 | B | GSTM1 | 204418_x_at | 2.00 | A |
| ESR1 | 207672_at | 2.00 | A | AI224578 | 235247_at | 1.00 | B | GSTM1 | 204550_x_at | 2.00 | A |
| ESR1 | 211233_x_at | 2.00 | A | AI283268 | 232579_at | 1.00 | B | GSTM1 | 215333_x_at | 2.00 | A |
| ESR1 | 211234_x_at | 2.00 | A | AP2B1 | 234064_at | 1.00 | B | GSTM3 | 202554_s_at | 1.00 | A |
| ESR1 | 211235_s_at | 2.00 | A | AW014921 | 230710_at | 1.00 | B | HER2 | 210930_s_at | 2.00 | A |
| ESR1 | 211627_x_at | 2.00 | A | AW014921 | 236480_at | 1.00 | B | HER2 | 216836_s_at | 2.00 | A |
| ESR1 | 215552_s_at | 2.00 | A | AYIL2 | 241511_at | 1.00 | B | HOXB13 | 209844_at | 4.00 | A |
| ESR1 | 217163_at | 2.00 | A | CDCA7 | 224428_s_at | 1.00 | B | HRASLS | 219983_at | 1.00 | A |
| ESR1 | 217190_x_at | 2.00 | A | COCA7 | 230060_at | 1.00 | B | HRASLS | 219984_s_at | 1.00 | A |
| EXT1 | 201995_at | 1.00 | A | COL4A2 | 237624_at | 1.00 | B | IDE | 2033328_x_at | 3,00 | A |
| EXT1 | 215206_at | 1.00 | A | DCK | 224115_at | 1.00 | B | FBXO31 | 223745_at | 1.00 | B |
| FBXO31 | 219784_at | 1.00 | A | DTL | 222680_s_at | 1.00 | B | FBXO31 | 224162_s_at | 1.00 | B |
| FBXO31 | 219785_s_at | 1.00 | A | EBF4 | 233032_x_at | 1.00 | B | FBXO31 | 236873_at | 1.00 | B |
| FBXO31 | 222352_at | 1.00 | A | EBF4 | 233850_s_at | 1.00 | B | FGF18 | 231382_at | 1.00 | B |
| FGF18 | 206986_at | 1.00 | A | ECT2 | 234992_x_at | 1.00 | B | FLT1 | 226497_s_at | 1.00 | B |
| FGF18 | 206987_x_at | 1.00 | A | ECT2 | 237241_at | 1.00 | B | FLT1 | 226498_at | 1.00 | B |
| FGF18 | 211029_x_at | 1.00 | A | EGLN1 | 223045_at | 1.00 | B | FLT1 | 232809_s_at | 1.00 | B |
| FGF18 | 211485_s_at | 1.00 | A | EGLN1 | 223046_at | 1.00 | B | GPR180 | 231871_at | 1.00 | B |
| FGF18 | 214294_s_at | 1.00 | A | EGLN1 | 224314_s_at | 1.00 | B | GPR180 | 232912_at | 1.00 | B |
| FLT1 | 204406_at | 1.00 | A | EXT1 | 232174_at | 1.00 | B | GSTM3 | 235867_at | 1.00 | B |
| FLT1 | 210287_s_at | 1.00 | A | EXT1 | 234634_at | 1.00 | B | LOC286052 | 241370_at | 1.00 | B |
| FLT1 | 222033_s_at | 1.00 | A | EXT1 | 237310_at | 1.00 | B | | | | |
| GMPS | 214431_at | 1.00 | A | EXT1 | 239227_at | 1.00 | B | | | | |
| GNAZ | 204993_at | 1.00 | A | EXT1 | 239414_at | 1.00 | B | | | | |
| GPR126 | 213094_at | 1.00 | A | EXT1 | 242126_at | 1.00 | B | | | | |

**Selection of genes to build the signature** .

A univariate Cox regression analysis was run to select genes whose expression levels were significantly correlated to Disease Free Survival (DFS). All genes correlated with DFS with a p<0.01 were selected: 48 genes satisfied this condition. Using an unsupervised hierarchical clustering algorithm 20 clusters were selected grouping genes with similar expression profiles. Or each cluster a representative gene was selected as the one most correlated with DFS within that specific cluster, using a Cox regression model.

The final 20 genes set was selected (Table 2).

Table 2: Final 20 genes set, all highly associated with Disease Free Survival (DFS)

| Index | Symbol | cluster | AffyID | Group | Group | Chip | Indice | P | HR | logP | logHR |
|---|---|---|---|---|---|---|---|---|---|---|---|
| 11 | AYTL2 | 5 | 201818_as | 1 | VV | A | 10 | 0.800828 | 1.461993 | 3.081881 | 0.379901 |
| 38 | MMPS | 14 | 203936_s_at | 1 | VV | A | 37 | 0.800607 | 1.147978 | 3.216767 | 0.138002 |
| 54 | PITRM1 | 17 | 205273_s_at | 1 | VV | A | 53 | 0.807143 | 1.285029 | 2.146102 | 0.258533 |
| 69 | TGFB3 | 3 | 209747_at | 1 | VV | A | 68 | 0.00688 | 0.793411 | 3.065539 | 0.231414 |
| 114 | PRC1 | 1 | 218009_s_at | 1 | VV | A | 113 | 7.17E-10 | 1.293216 | 9.144529 | 0.257132 |
| 120 | ORC6L | 16 | 219106_x_at | 1 | VV | A | 118 | 0.000201 | 1.437993 | 3.896472 | 0.383288 |
| 126 | FBXO31 | 7 | 219785_s_at | 1 | VV | A | 125 | 0.004459 | 1.364903 | 2350769 | 0.311083 |
| 143 | MS4A7 | 15 | 223344_s_at | 1 | VV | B | 142 | 0.004351 | 0.849666 | 2.381449 | 0. 162911 |
| 145 | SERF1A | 19 | 223539_s_at | 1 | VV | B | 144 | 0.001192 | 1.438202 | 2.923742 | 0.363394 |
| 156 | QSOX2 | 18 | 227146_at | 1 | VV | B | 155 | 0.003409 | 1.661511 | 2.467395 | 0.507728 |
| 163 | FGF18 | 8 | 231382_at | 1 | VV | B | 162 | 0.003375 | 0.666622 | 2.471695 | 0.405532 |
| 164 | GPR180 | 8 | 231871_at | 1 | VV | B | 163 | 0.005603 | 1.386805 | 2.251818 | 0.327002 |
| 66 | BOL2 | 6 | 203695_at | 2 | Paik | A | 32 | 0.00331 | 8.853341 | 2.4502 | -0.158596 |
| 7 | IGFBP7 | 4 | 201163_s_at | 3 | IR | A | 6 | 0.001529 | 0.661036 | 2.815582 | -0.413869 |
| 26 | IDE | 11 | 203328_x_at | 3 | IR | A | 25 | 0.035188 | 1.578935 | 2.284982 | 0.51816 |
| 35 | IGFBP6 | 2 | 203951_at | 3 | IR | A | 34 | 2.22E-06 | 0.671623 | 5.654231 | -0.398058 |
| 45 | IRS1 | 13 | 204636_at | 3 | IR | A | 44 | 0.001258 | 0.624991 | 2.900183 | -0.192383 |
| 47 | IL6ST | 12 | 204963_s_at | 3 | IR | A | 46 | 2.77E-05 | 0.82816 | 4.556887 | -0.188549 |
| 68 | IGF1 | 2 | 208540_at | 3 | IR | A | 67 | 1.03E-06 | 0.801831 | 5.985837 | -0.220858 |
| 102 | TNFSF10 | 20 | 214329_x_at | 3 | IR | A | 101 | 0.004448 | 0.822796 | 2.351829 | -0.195047 |

**Tumor Samples and Signature creation on the training set**

Among 350 consecutive invasive breast cancer patients with full information about tumor, adjuvant treatments, follow up, relapse, death and causes of death, treated between 1998 and 2001, 89 cases (25.4%) were removed from the study because of the low RNA concentration (below 10 ng/$\mu$L) or high degradation (*Ct* values for *ACTB* and *B2M* over 34). The remaining 261 cases were split in two biological sample datasets: The training (137 cases) and the validation set (124 cases) by a simple criteria of consecutiveness. The clinical and demographic characteristics of the patients included in the training and in the validation set are summarized in Table 3 and reported in detail in the supplementary file. Due to a simple criteria of consecutiveness building the sets, the Training set has a longer mean follow up (100.7 months; range 59-123) as compared with the Validation set (89.2; 61-121). Nevertheless, the only significant differences between the two sets was the use of anthracycline-based regimens in the adjuvant setting (Training 16% *vs.* Validation 32.2%; *p* = 0.01) and an higher incidence of G3 tumors in the Validation Set (30.6% *vs.* 19.7, *p* = 0.04). The lack of information about HER2 Status is related to the temporal context of the selected cases (1998-2001) and it was evaluated "*a posteriori*" just in 40% of relapsed patients. Any other clinical and biological pattern is similar and reflecting the "real life" picture of the disease in North East of Italy at this time.

**Table 3.** Characteristics of patients and tumors in the Training and Validation sets.

| | Training Set | | Validation Set | | *p* value |
|---|---|---|---|---|---|
| **Nr of Patients** | 137 | | 124 | | ns |
| **Mean Age (range)** | 62.3 (35-87) | | 61.1 (33-87) | | ns |
| **Mean Follow up (mouths)** | 100.7 (59-123) | | 89.2 (61-121) | | ns |
| **Histology** | *n* | % | *n* | % | *p* value |
| Ductal | 86 | 62.8 | 83 | 66.9 | ns |
| Lobular | 26 | 19 | 16 | 12.9 | ns |
| Tubular-Lobular | 12 | 8.8 | 10 | 8.5 | ns |
| Medullary/Apoerine | 2 | 1.4 | 3 | 2.4 | ns |
| Other | 11 | 8.02 | 12 | 9.6 | ns |
| **T Size** | | | | | |
| T1 | 78 | 56.9 | 82 | 66.1 | ns |
| T2 | 53 | 38.7 | 37 | 29.8 | ns |
| T3 | 3 | 2.2 | 3 | 2.4 | ns |
| Tx | 3 | 2.2 | 2 | 1.6 | ns |
| **N Status** | | | | | |
| pN0 | 89 | 65 | 75 | 60.5 | ns |
| pN1a | 26 | 19 | 26 | 21 | ns |
| pN+ 4-10 | 11 | 8.1 | 7 | 5.6 | ns |
| pN+ >10 | 10 | 7.3 | 14 | 11.3 | ns |
| NX | 0 | | | | |
| ER/PgR pos | 123 | 85.4 | 97 | 76.38 | ns |
| HER2 NA | 125 | 91.2 | 79 | 73.7 | *p* = 0.05* |
| **Grading** | | | | | |
| G1 | 33 | 24.1 | 20 | 16.1 | ns |
| G2 | 51 | 37.2 | 57 | 46 | ns |
| G3 | 27 | 19.7 | 38 | 30.6 | *p* = 0.04 |
| G NA | 26 | 19 | 9 | 7.3 | ns |
| **Ki67** | | | | | |
| High (>14%) | 60 | 43.8 | 60 | 48.4 | Ns |
| Low (<15%) | 77 | 56.2 | 60 | 48.4 | ns |
| Adjuvant Chemo | 49 | 35.8 | 57 | 46 | ns |
| Anthracycline-based | 22 | 16 | 40 | 32.2 | *p* = 0.01 |
| Adjuvant endocrine (any) | 110 | 80.3 | 96 | 77.4 | *p* = 0.01 |
| Relapses | 33 | 24 | 38 | 30.6 | ns |

(continued)

| Ki67 | | | | | |
|---|---|---|---|---|---|
| Mean DFS, months | 51.4 | | 47.2 | | ns |
| Deaths | 33 | 24 | 39 | 31.4 | ns |

* In the Validation Set HER2 status was evaluated in relapsed patients.

The biological samples dataset was split by a random procedure into a training and a validation set (ratio 1:1). The gene signature was developed on the training set. Once the signature has been fully specified, the validation set was accessed once and only for estimating the prediction accuracy of the signature. The training set was made up of 144 samples of breast cancer with complete prognosis information (mean follw up = 7.5 years). The RealTime PCR gene expression values have been normalized by GeNorm using 2 housekeeping genes (B2M e ACTB).

The normalized expression levels were entered into a multivariate Cox regression model with a stepwise selection procedure. The Cox model selected the genes more correlated with DFS and created the signature. Two selection criteria were used, the first one more conservative and less discriminate, the second one less conservative but more discriminant. The first signature was based on the 5 genes more correlated with DFS, while the second one was based on the 11 genes more correlated with DFS.

Their expression levels were combined in a weighted linear score $i$

$$GeneSignatureScore \sum_i wivalue_i$$

where wi are weights determined by the coefficients of the multivariate Cox regression model (B values in the Table 4) and valuei are the gene expression levels. The tertile values of the signature were chosen as the cut offs to classify patients at low, medium or high risk of disease relapse.

11 genes

**Variables in the Equation**

| | B | SE | Wald | df | Sig. | Exp(B) | 96.0% CI for Exp(B) | |
|---|---|---|---|---|---|---|---|---|
| | | | | | | | Lower | Upper |
| fgf18 | .202 | .078 | 6.679 | 1 | .010 | 1.224 | 1.050 | 1.426 |
| bcl2 | -847 | .243 | 12.128 | 1 | .000 | .429 | .266 | .690 |
| ide | .389 | .226 | 2.962 | 1 | .085 | 1.475 | .947 | 2.296 |
| osox2 | -.162 | .140 | 1.681 | 1 | .195 | .834 | .633 | 1.098 |
| patron1 | -343 | .209 | 2.707 | 1 | .100 | .709 | .471 | 1.068 |
| prc1 | .550 | .160 | 11.844 | 1 | .001 | 1.734 | 1.267 | 2.372 |
| mmp9 | .123 | .071 | 3.016 | 1 | .082 | 1.131 | .934 | 1.300 |
| igfbp8 | -.184 | .090 | 4.236 | 1 | .040 | .832 | .698 | .991 |
| irs1 | .189 | .142 | 1.781 | 1 | .182 | 1.208 | .916 | 1.594 |
| Inst | .163 | .114 | 2.923 | 1 | .155 | 1.176 | .940 | 1.472 |
| serf1a | -262 | .086 | 9.336 | 1 | .002 | .769 | .650 | .910 |

A multivariate stepwise Cox analysis was run on the breast cancer samples including the 20 selected genes. The Cox model selected a final set of five genes independently associated with DFS

(Table 5, HR: Hazard Ratio): *FGF18* (HR = 1.13, $p$ = 0.05), *BCL2* (HR = 0.57, $p$ = 0.001), *PRC1* (HR = 1.51, $p$ = 0.001), *MMP9* (HR = 1.11, $p$ = 0.08), *SERF1a* (HR = 0.83, $p$ = 0.007).

These five genes were combined into a linear score (signature) weighted according to the coefficients of the Cox model (Table 5), as:

$$\text{Gene Signature Score} = 0.125 \times FGF18 - 0.560 \times BCL2 + 0.409 \times PRC1 + 0.104 \times MMP9 - 0.188 \times SERF1a$$

This score ranged from -2.95 to 2.91, with a mean value of -0.48 a SD of 1.00. The linear score was highly associated with DFS in the training set: HR = 2.7, 95% CI = 1.9-4.0, $p < 0.001$.

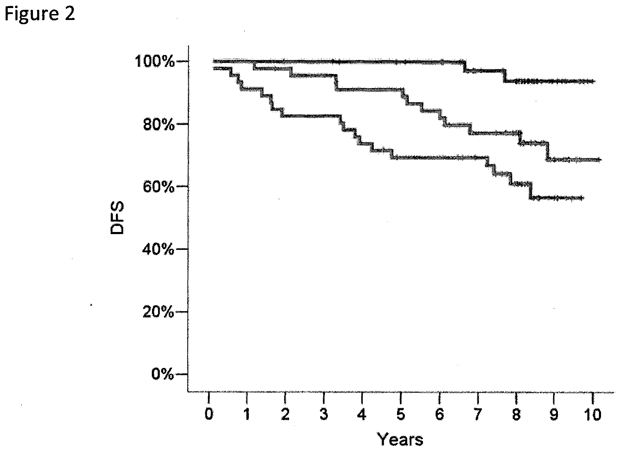

The score was then categorized in three groups according to the tertiles of its distribution. The DFS according to the three risk groups is reported in Figure 2: Patients with an intermediate risk signature had an HR = 6.03, (95% CI = 1.35-27.0, $p = 0.019$) and patients with a high risk signature had an HR = 10.8, (95% CI = 2.51-46.64, $p = 0.001$) as compared to patients with a low risk signature.

Table 5

| | | | | | | | 95.0% CI for Exp(B) | |
|---|---|---|---|---|---|---|---|---|
| | B | SE | Wald | df | Sig. | Exp(B) | Lower | Upper |
| lgf18 | .125 | .064 | 3.738 | 1 | .053 | 1.133 | .998 | 1.285 |
| bcl2 | -650 | .173 | 10.444 | 1 | .001 | .571 | .407 | .802 |
| prc1 | .409 | .120 | 11.712 | 1 | .001 | 1.505 | 1.191 | 1.903 |
| mmp9 | -104 | .060 | 3.031 | 1 | .082 | 1.109 | .987 | 1.247 |
| serf16 | -188 | .069 | 7.375 | 1 | .007 | .828 | .723 | .949 |

*Variables in the Equation*

The correlation of the two signatures with DFS was evacuate by Kaplan Meier (KM) survival curves and by the Hazard estimate by a Cox model (Figure 2). Figure 2 shows Probability of 5 years relapse: Disease free survival (DFS) according to the risk groups defined by the gene signature in the training set: Low risk group (first curve from the top), intermediate risk group (middle curve), high risk group (third curve from the top). The hazard ratio (HR) of DFS for intermediate risk patients as compared to low risk is 6.0 (95% Confidence Intervals (CI) = 1.35-27.0, $p = 0.019$ and the HR of DFS for high risk patients as compared to low risk is 10.8 (95% CI = 2.51-46.6, $p = 0.001$).

**Signature validation on the validation set**

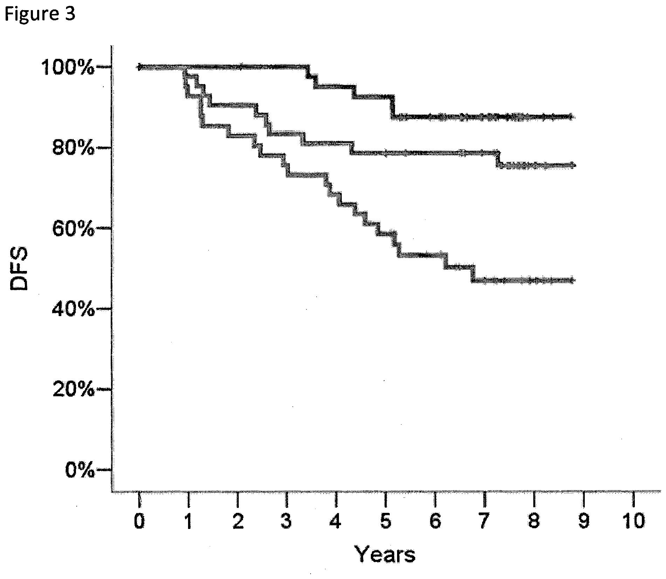

The prognostic value of the signature was tested on the validation set made up of 127 breast cancer samples of which 37 were recurrences, by Kaplan Meier survival analysis and Cox regression analysis. The discrimination ability of the signature was assessed on the validation set by a Kaplan Meier analysis, using the same cut offs classifying patients at low, intermediate or high risk of disease relapse as defined on the training set. The score resulted highly associated with DFS also in the validation set ($p < 0.001$) (Figure 3). Patients with an "intermediate risk" signature had an HR = 2.1 (95% CI = 0.72-6.2, $p = 0.17$) and patients with a high risk signature had an HR = 5.4 (95% CI = 2.0-14.4, $p = 0.001$) as compared to patients with a low risk signature.

Figure 3 shows Validation set: Probability of 5 years relapse. Disease free survival (DFS) according to the risk groups defined by the gene signature in the validation set: low risk group (first curve from the top), intermediate risk group (middle curve), high risk group (third curve from the top). The hazard ratio (HR) of DFS for intermediate risk patients as compared to low risk is 2.1 (95% Confidence Intervals (CI) = 0.72-6.2, $p = 0.17$) and the HR of DFS for high risk patients as compared to low risk is 5.4 (95% CI = 2.0-14.4, $p = 0.001$).

**Inter and Intra Assay Reproducibility**

Three serial sections from three cases each were evaluated independently in triplicate calculating the coefficients of variation (CVs) for the Recurrent Score in the same run and in different runs. The intra-assay and the inter-assay CVs was 3.7% and 4.7%, respectively.

**Univariate Analysis**

In the Univariate Analysis variables significantly related to DFS were Nodal Status ($p = 0.0000001$), T Size ($p = 0.000002$), the five gene Signature ($i = 0.000043$), Ki67 ($p = 0.0007$) and Grading ($p = 0.027$) (Table 6).

Table 6: Univariate analysis

| Variable | Regression coefficient (B) | SE | Exp (B) | Mean | Z-value | Probability level |
|---|---|---|---|---|---|---|
| Nodal Status (pN0/pN1a/pN2)· | 0.591 | 0.100 | 1.806 | 0.062 | 5.1 | 0.0000001 |
| T Size (pT1/pT2/pT3) | 3.647 | 7.639 | 1.037 | 20.195 | 4.77 | 0.000002 |
| 5 gene Signature (High/ Intermediate/Low) | 0.646 | 0.158 | 1.909 | 1.984 | 4.09 | 0.000043 |
| ki67 (High/Low) | 0.427 | 0,126 | 1.533 | 1.933 | 3.38 | 0,0007 |
| Grading (G1/G2/G3) | 0.298 | 0.135 | 1.348 | 1.798 | 2.2 | 0.027 |

**Multivariate Analysis**

The Multivariate Analysis (Cox Regression) indicates that Nodal Status ($p$ = 0.00001), T Size ($p$ = 0.0002) and the five-gene Signature ($p$ = 0.0004) are significantly related to DFS, while Ki67 (cut off: 14%), Grading and Chemo- or Endocrine Adjuvant Treatments are not (Table 7). The five-gene Signature HR is slightly affected by adjuvant treatments: Table 8 summarized data about the five-gene signature in presence or absence of Adjuvant treatment.

Table 7: multivariate Cox regression analysis

| Variable | Regression coefficient (B) (95% CI) | SE | Exp (B) | Mean | Z-value | Probability level |
|---|---|---|---|---|---|---|
| Nodal Status (pN0/pN1a/pN2) | 0.551 (0.350-0.752) | 0.102 | 1.736 | 0.655 | 5.379 | 0.00001 |
| T Size (pT1/pT2/pT3) | 0.562 (0.269-0.854) | 0.149 | 1.754 | 1.449 | 3.762 | 0.0002 |
| 5 gene Signature (High/ Intermediate/Low) | 0.666 (0.298-1.034) | 0.187 | 1.947 | 1.9767 | 3.549 | 0.0004 |
| Ki67 (High/Low) | 0.27 (-0,028-0.569) | 0.152 | 1.31 | 1.748 | 1.77 | 0.076 |
| Grading (G1/G2/G3) | -0.111 (-0.387-0.164) | 0.14 | 0.894 | 1.798 | -0.792 | 0.428 |
| AdjChemo (Yes/No) | 0.061 (-0.479-0.601) | 0.275 | 1.063 | 1.604 | 0.221 | 0.824 |
| Adj Endocrine (Yes/No) | 0.032 (-0.556-0.622) | 0.3 | 1.033 | 1.209 | 0.109 | 0.912 |

Table 8: Hazard Ratio Longrank (Cox-Mantel) for five genes of the invention in presence or absence of adjuvant treatments

| Chemo or endocrine adjuvant treatment | | | | | | |
|---|---|---|---|---|---|---|
| | | YES | | | NO | |
| 5 Gene Score | HR | 95% CI | $p$ value | HR | 95% CI | $p$ value |
| Low *vs*. High | 0.35 | 0.20-0.60 | 0.0006 | 0.16 | 0.08-0.32 | 0.0001 |
| Low vs. Intermediate | 0.98 | 0.45-2.11 | 0.9 | 0.29 | 0.11-0.77 | 0.0224 |
| Intermediate *vs*. High | 0.4 | 0.23-0.69 | 0.002 | 0.56 | 0.29-1.06 | 0.089 |

The above description shows in detail the steps to achieve the method of the present invention. The method for predicting the likelihood of long-term survival of a breast cancer patient without the recurrence of breast cancer, following surgical removal of the primary tumor, comprising analysis of the expression level of the following 5 mRNA including: *FGF18, BCL2, PRC1, MMP9, SERF1a,* wherein overexpression of *BCL2 and SERF1a,* indicates a decreased likelihood of longterm survival without breast cancer recurrence, and the overexpression of *FGF18, PRC1 andMMP9,* indicates an increased likelihood of long-term survival without breast cancer recurrence.

This method has a good discriminating ability when tested on the validation set and will be proposed as a prognostic signature for DFS (disease free survival) in breast cancer patients.

The method disclosed herein may be carried out on total RNA isolated from paraffin embedded, formalin fixed (FFPE) tissue specimen of said patient. Moreover, the method may be performed in a sample wherein said total RNA is isolated from a tissue area in which there are at least 70% of cancer cells. The RNA may be fragmented RNA. The RNA may be isolated from a fine needle biopsy sample. The RNA may be isolated from fresh/frozen tissue. The method may further comprise creating a report summarizing the data obtained by the gene expression analysis by RT-qPCR (reverse transcriptase-quantitative polymerase chain reaction). The report may include prediction of the likelihood of long term survival of said patient without the recurrence of breast cancer following surgical removal of the primary tumor. The report may include recommendation for a treatment modality of said patient. The two or more housekeeping genes may be selected from the group consisting of *ACTB, GAPDH, B2M, TBP.* The method may further comprise identifying a treatment option for the patient based on the normalized expression level. The expression level of the *FGF18, BCL2, PRC1, MMP9,SERF1a* transcripts, or its expression products, may be normalized against a reference set comprising RNA transcripts of two or more housekeeping genes, or their expression products by GeNorm and the statistical computing language *R*. The method may determine a normalized expression level of the RNA transcript of *FGF18, BCL2, PRC1, MMP9, SERF1a* or its expression product, wherein the normalized expression level of the RNA transcript or its expression product allows the calculation of the following algorithm:

$$GeneSignatureScore = 0.125 \text{x} FGF18 - 0.560 \text{x} \; BCL2 + 0.409 \text{x} \; PRC1 + 0.104 \text{x} \; MMP9 - 0.188 \; \text{x} SERF1$$

The *GeneSignatureScore* may categorize 3 groups according to the tertiles of its distribution:

Low Risk of recurrence within 5 years: *GeneSignatureScore* <-0.87
Intermediate Risk of recurrence within 5 years: -0.87 < *GeneSignatureScore* < -0.13
High Risk of recurrence within 5 years: *GeneSignatureScore* >=-0.13

The method may further comprise (a) assaying an expression level of at least one RNA transcript or its expression product in a biological sample, fine needle aspirate, either fresh frozentissue or formalin fixed paraffin embedded tissue (FFPE) comprising at least one breast cancer cell obtained from the patient, wherein the at least one RNA transcript is the transcript of a gene selected from the group consisting of: *FGF18, BCL2, PRC1, MMP9, SERF1*; (b) determining a normalized expression level of the RNA transcript or its expression product wherein the normalized expression level of the RNA transcript or its expression product positively correlates with an increased likelihood of breast cancer recurrence; and (c) providing information comprising the likelihood of long-term survival without breast cancer recurrence for the patient, wherein the information comprises the normalized expression level of the RNA transcript or its expression product.
The present invention is better illustrated by the examples, herein below, which in no way assume limiting value.

**Experimental Section**

1. Tumor Samples Enrolled in This Study Tumor samples were obtained from routinely processed formalin-fixed, paraffin embedded sections retrieved from 350 consecutive invasive breast cancer patients with full information about tumor, adjuvant treatments, follow up, relapse, death and causes of death, treated between 1998 and 2001. In order to test signature in a "real life" clinical setting, it is used consecutive non metastatic breast cancer cases irrespectively from age, nodal and estrogen receptor status, adjuvant treatment. The only requested pattern was a minimum follow up of 5 years with full clinical data. All patient information was handled in accordance with review board approved protocols and in compliance with the Helsinki declaration. Hematoxylin and Eosin (H & E) sections were reviewed to identify paraffin blocks with tumor areas. Histological type and grade were assessed according to the World Health Organization criteria. The detailed histological and clinical feature of each patient enrolled in this study is available in the supplementary information file. Paraffin blocks corresponding to histology sections that showed the highest relative amount of tumor vs. stroma, few infiltrating lymphoid cells and that lacked significant areas of necrosis were selected. Three 20 μm thick sections were cut, followed by one H & E control slide. The tumor area selected for the analysis was marked on this control slide to ensure greater than 70% content of neoplastic cells. Tumor areas dissected ranged from 0.5 to 1.0 cm2 wide.

2. Ethics Statement

The use of tissues for this study has been approved by the Ethics Committee of Centro Oncologico, ASS1 triestina & Università di Trieste, Italy. A comprehensive written informed consent was signed for the surgical treatment that produced the tissue samples and the related diagnostic procedures. All information regarding the human material used in this study was managed using anonymous numerical codes, clinical data were not used and samples were handled in compliance with the Helsinki declaration (http://www.wma.net/en/30publications/10policies/b3/).

### 3. Gene Expression Analysis on Breast Cancer Samples

#### 3.1. RNA Isolation

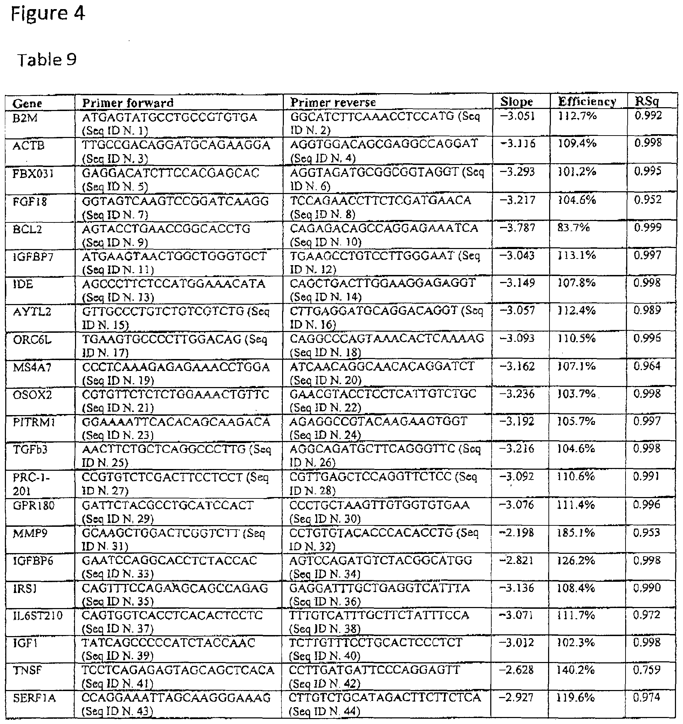

Paraffin-embedded tumor material obtained from the 20 $\mu$m thick sections was de-paraffinized in xilene at 50 °C for 3 min and rinsed twice in absolute ethanol at room temperature. Total RNA was extracted using the RecoverAll kit (Ambion, Austin, TX, USA), including a DNase step according to the manufacturer's recommended 3.3.2. Primers Design Primers were designed using Primer3 software (http://simgene.com/Primer3) and are described in Figure 4. Amplicons were tested by *MFOLD* (http://mfold.rna.albany.edu/?q=mfold) in order to avoid secondary structures within primer positions and they were tested by repeatmasker (http://www.repeatmasker.org) and primer-BLAST (http://www.ncbi.nlm.nih.gov/tools/primer-blast) for primer specificity.

**Figure 4 .** Primer sequences, slope, PCR efficiency and RSq of each of the 20 genes + 2 housekeeping genes.

3.3. Two Step qRT-PCR Analysis Fourteen $\mu$L of total RNA was subjected to reverse transcription using SuperScript® VILO™ cDNA Synthesis kit (Invitrogen, Carlsbad, CA, USA) according to the manufacturer's recommended protocol. One microlitres of cDNA was amplified in duplicate adding 10 picomoles of each primer (see Figure 4 for sequence details) to the 1x QuantiFast™ SYBR® Green PCR solution (Qiagen, Hilden, Germany) in a final volume of 25 $\mu$L.

Cycling conditions consisted of 5 min at 95 °C, 10 s at 95 °C, 30 s at 60 °C for a total of 40 cycles, using Stratagene Mx3000™ or ABI SDS 7000™ instruments. Plate reading was performed during the 60 °C step.

For each primer set, standard curves made from serial dilutions of cDNA from MCF7 cell lines (see Table 3) were used to estimate PCR reaction efficiency (E) using the formula:

E (%) = (10[-1/sl°pe] - 1) $\times$ 100. The expression levels of each of the 20 genes selected were normalized by *GeNorm* using 2 housekeeping genes (*B2M* e *ACTB*) and the relative quantification was calculated by the statistical computing language *R*. The human breast cancer cell line MCF7 was purchased from American Type Culture Collection (ATCC HTB22; derived from a human breast adenocarcinoma). Cells were maintained in minimal essential medium (MEM) (Invitrogen/Life technologies, Villebon-sur-Yvette, France) supplemented with 2 mM L-glutamine, 1.5 g/L sodium bicarbonate, 0.1 mM nonessential aa, 1 mM pyruvate sodium, 0.01 mg/mL bovine insulin, and 10% fetal bovine serum (Thermo Scientific, Waltham, MA, USA) at 37 °C in a humidified atmosphere of 5% $CO_2$.

#### 3.4. Training and Validation Dataset

The biological samples dataset was split into the training and the validation dataset. The training set consists of the first 144 consecutive cases and the validation of the last 127 cases. The gene signature was developed on the training set. Once the signature has been fully specified, the validation set was accessed once and only for estimating the prediction accuracy of the signature. A multivariate stepwise Cox analysis was run on the breast cancer training set samples including the 20 selected genes. The stepwise procedure was run to select genes independently associated with DFS (*p* for inclusion <0.10).

The overall workflow shown in Figure 1 summarizes every step starting from selection of markers from the literature since the validation of the gene signature. Reproducibility within and between blocks was assessed by performing the test in serial sections from three blocks representing three cases. It is performed a multivariate Cox proportional-hazards analysis in a model that included treatment received (no adjuvant therapy vs. chemotherapy, hormonal therapy, or both) and the final gene Signature (both Training and Validation sets included), using the NCSS 2001 Statistical software (NCSS Inc., Kaysville, UT, USA, 2001).

#### 3.5. Univariate and Multivariate Analysis

It is performed a univariate analysis including Age, T size, Nodal status, Grading, Ki67, adjuvant treatments and the 5-gene signature, followed by a multivariate Cox proportional-hazards analysis in a model that included treatment received (no adjuvant therapy vs. chemotherapy, hormonal therapy, or both) and the 5-gene Signature (Low/Intermediate/High Risk; both Training and Validation sets included), using the NCSS 2001 Statistical software (NCSS Inc., Kaysville, UT, USA, 2001).

## Claims

1. A process for determining the prognosis of a patient suffering from breast cancer, comprising the step of measuring the level of expression of the following 5 mRNA including: FGF18, BCL2, PRC1, MMP9, SERF1a genes, in an isolated biological sample of said patient consisting of a tumour or a tumour biopsy, or neoplastic cells, or paraffin embedded tumour or formalin fixed tissue specimen (FFPE) containing tumour sample of said patient wherein a high BCL2 and SERF1a expression is associated with a poor prognosis and a decreased likelihood of long-term survival without breast cancer recurrence, and high FGF18, PRC1 and MMP9 expression is associated with a good prognosis and an increased likelihood of long-term survival without breast cancer recurrence, following surgical removal of the primary tumor.

2. The process according to claim 2, wherein said isolated biological sample is paraffin embedded tumor, formalin fixed tissue specimen (FFPE) containing sample of said patient.

3. The process according to any of claims 1 to 3 wherein said isolated biological sample contains at least 70% of cancer cells.

4. The process according to claim 1, wherein said measure of the expression level of FGF18, BCL2, PRC1, MMP9, SERF1a genes is determined by quantification of the expression of mRNA which encodes for of FGF18, BCL2, PRC1, MMP9, SERF1a proteins.

5. The process according to any of claims 1 to 4, wherein the expression level of FGF18, BCL2, PRC1, MMP9,SERF1a genes is determined by quantitative RT-PCR.

6. The process of claim 1, further comprising:

    (a) assaying an expression level of RNA transcripts of the group consisting of: FGF18, BCL2, PRC1, MMP9, SERF1a genes,
    (b) determining normalized expression levels of the RNA transcripts, wherein the normalized expression levels of the RNA transcripts correlate with the likelihood of breast cancer recurrence,
(c) providing information comprising the likelihood of long-term survival without breast cancer recurrence for the patient, wherein the information comprises the normalized expression level of the RNA transcript.

7. Use of level of expression for the following 5 mRNA including: FGF18, BCL2, PRC1, MMP9, SERF1a for determining a prognosis for the likelihood of long-term survival without breast cancer recurrence based on an isolated biological sample from a patient previously affected by breast cancer and following surgical removal; where such sample consists of a tumour or a tumour biopsy, or neoplastic cells, or paraffin embedded tumour or formalin fixed tissue specimen (FFPE) containing tumour sample of said patient.

**Patentansprüche**

1. Verfahren zum Bestimmen der Prognose einer an Brustkrebs leidenden Patientin, umfassend den Schritt zum Messen des Expressionsniveaus der folgenden 5 mRNA, umfassend: FGF18-, BCL2-, PRC1-, MMP9-, SERF1a-Gene, in einer isolierten biologischen Probe der Patientin, die aus einem Tumor oder einer Tumorbiopsie, oder neoplastischen Zellen, oder in Paraffin eingebettetem Tumor oder in Formalin fixierter Gewebeprobe (FFPE), die eine Tumorprobe der Patientin enthält, besteht, wobei eine hohe BCL2- und SERF1a-Expression mit einer mangelnden Prognose und einer geringen Wahrscheinlichkeit des langfristigen Überlebens ohne Rückfall des Brustkrebses assoziiert ist, und eine hohe FGF18-, PRC1- und MMP9-Expression mit einer guten Prognose und einer erhöhten Wahrscheinlichkeit des langfristigen Überlebens ohne Rückfall des Brustkrebses assoziiert ist, nach der operativen Entfernung des Primärtumors.

2. Verfahren nach Anspruch 2, in dem die isolierte biologische Probe ein in Paraffin eingebetteter Tumor ist, in Formalin fixierte Gewebeprobe (FFPE), welche die Probe der Patientin enthält.

3. Verfahren nach einem der Ansprüche 1 bis 3, in dem die isolierte biologische Probe mindestens 70% von Krebszellen enthält.

4. Verfahren nach Anspruch 1, in dem die Messung des Expressionsniveaus der FGF18-, BCL2-, PRC1-, MMP9-, SERFla-Gene durch Quantifizierung der Expression der mRNA bestimmt wird, die für die FGF18-, BCL2-, PRC1-, MMP9-, SERF1a-Proteine kodiert.

5. Verfahren nach einem der Ansprüche 1 bis 4, in dem das Expressionsniveau der FGF18-, BCL2-, PRC1-, MMP9-, SERFla-Gene durch quantitative RT-PCR bestimmt wird.

6. Verfahren nach Anspruch 1, außerdem umfassend:

(a) Analysieren eines Expressionsniveaus der RNA-Transkripte aus der Gruppe, bestehend aus: FGF18-, BCL2-, PRC1-, MMP9-, SERFla-Genen,
(b) Bestimmen normalisierter Expressionsniveaus der RNA-Transkripte, wobei die normalisierten Expressionsniveaus der RNA-Transkripte mit der Wahrscheinlichkeit des Rückfalles des Brustkrebses verknüpft sind,
(c) Liefern von Informationen, welche die Wahrscheinlichkeit des langfristigen Überlebens ohne Rückfall des Brustkrebses für die Patientin umfassen, wobei die Informationen das normalisierte Expressionsniveau des RNA-Transkripts umfassen.

7. Verwendung des Expressionsniveaus für die folgenden 5 mRNA, umfassend: FGF18, BCL2, PRC1, MMP9, SERF1a zum Bestimmen einer Prognose für die Wahrscheinlichkeit des langfristigen Überlebens ohne Rückfall des Brustkrebses anhand einer isolierten biologischen Probe einer zuvor von Brustkrebs betroffenen Patientin und nach der operativen Entfernung; wobei die Probe aus einem Tumor oder einer Tumorbiopsie, oder neoplastischen Zellen, oder in Paraffin eingebettetem Tumor oder in Formalin fixierter Gewebeprobe (FFPE), welche die Tumorprobe der Patientin enthält, besteht.

**Revendications**

1. Procédé pour déterminer le pronostic d'une patiente souffrant de cancer du sein, comprenant l'étape consistant à mesurer le niveau d'expression des 5 ARNm suivantes, incluant : gènes FGF18, BCL2, PRC1, MMP9, SERFla, dans an échantillon biologique isolé de ladite patiente consistant en une tumeur ou une biopsie tumorale, ou des cellules néoplasiques, ou une tumeur inclue dans la paraffine ou un échantillon de tissu fixé au formol (FFPE), qui contient un échantillon de tumeur de ladite patiente, dans lequel une expression élevée de BCL2 et SERF1a est associée avec un pronostic sombre et une probabilité réduite de survie à long terme sans récidives du cancer du sein, et une expression élevée de FGF18, PRC1 et MMP9 est associée avec un bon pronostic et une probabilité augmentée de survie à long terme sans récidives du cancer du sein, après l'ablation chirurgicale de la tumeur primaire.

**2.** Procédé selon la revendication 2, dans lequel ledit échantillon biologique isolé est une tumeur inclue dans la paraffine, un échantillon de tissu fixé au formol (FFPE), qui contient un échantillon de ladite patiente.

**3.** Procédé selon l'une quelconque des revendications 1 à 3, dans lequel ledit échantillon biologique isolé contient au moins 70% de cellules cancéreuses.

**4.** Procédé selon la revendication 1, dans lequel ladite mesure du niveau d'expression de gènes FGF18, BCL2, PRC1, MMP9, SERF1a est déterminée par une quantification de l'expression de ARNm qui code pour les protéines FGF18, BCL2, PRC1, MMP9, SERF1a.

**5.** Procédé selon l'une quelconque des revendications 1 à 4, dans lequel le niveau d'expression des gènes FGF18, BCL2, PRC1, MMP9, SERF1a est déterminé par RT-PCR quantitative.

**6.** Procédé selon la revendication 1, comprenant de plus :

(a) tester un niveau d'expression de transcrits ARN du groupe consistant en : gènes FGF18, BCL2, PRC1, MMP9, SERFla,
(b) déterminer les niveaux d'expression normalisés des transcrits ARN, les niveaux d'expression normalisés des transcrits ARN étant en corrélation avec la probabilité de récidive du cancer du sein,
(c) fournir des informations comprenant la probabilité de survie à long terme sans récidives du cancer du sein pour la patiente, dans lequel les informations comprennent le niveau d'expression normalisé du transcrit ARN.

**7.** Utilisation du niveau d'expression pour les 5 ARNm suivantes, incluant : FGF18, BCL2, PRC1, MMP9, SERF1a pour déterminer un pronostic pour la probabilité de survie à long terme sans récidives du cancer du sein au moyen d'un échantillon biologique isolé d'une patiente précédemment atteinte d'un cancer du sein et après l'ablation chirurgicale ; ledit échantillon consistant d'une tumeur ou une biopsie tumorale, ou des cellules néoplasiques, ou une tumeur inclue dans la paraffine ou un échantillon de tissu fixé au formol (FFPE), qui contient un échantillon de tumeur de ladite patiente.

## Figure 1

**Figure 1.** Construction of the gene-set predictor/gene signature for risk prediction.
(A) Gene selection on the published datasets; (B) Gene selection on the merged Gene Expression Omnibus (GEO) datasets; (C) Developing the gene signature:.

Figure 2

Figure 3

# Figure 4

## Table 9

| Gene | Primer forward | Primer reverse | Slope | Efficiency | RSq |
|------|----------------|----------------|-------|-----------|-----|
| B2M | ATGAGTATGCCTGCCGTGTGA (Seq ID N. 1) | GGCATCTTCAAACCTCCATG (Seq ID N. 2) | −3.051 | 112.7% | 0.992 |
| ACTB | TTGCCGACAGGATGCAGAAGGA (Seq ID N. 3) | AGGTGGACAGCGAGGCCAGGAT (Seq ID N. 4) | −3.116 | 109.4% | 0.998 |
| FBX031 | GAGGACATCTTCCACGAGCAC (Seq ID N. 5) | AGGTAGATGCGGCGGTAGGT (Seq ID N. 6) | −3.293 | 101.2% | 0.995 |
| FGF18 | GGTAGTCAAGTCCGGATCAAGG (Seq ID N. 7) | TCCAGAACCTTCTCGATGAACA (Seq ID N. 8) | −3.217 | 104.6% | 0.952 |
| BCL2 | AGTACCTGAACCGGCACCTG (Seq ID N. 9) | CAGAGACAGCCAGGAGAAATCA (Seq ID N. 10) | −3.787 | 83.7% | 0.999 |
| IGFBP7 | ATGAAGTAACTGGCTGGGTGCT (Seq ID N. 11) | TGAAGCCTGTCCTTGGGAAT (Seq ID N. 12) | −3.043 | 113.1% | 0.997 |
| IDE | AGCCCTTCTCCATGGAAACATA (Seq ID N. 13) | CAGCTGACTTGGAAGGAGAGGT (Seq ID N. 14) | −3.149 | 107.8% | 0.998 |
| AYTL2 | GTTGCCCTGTCTGTCGTCTG (Seq ID N. 15) | CTTGAGGATGCAGGACAGGT (Seq ID N. 16) | −3.057 | 112.4% | 0.989 |
| ORC6L | TGAAGTGCCCCTTGGACAG (Seq ID N. 17) | CAGGCCCAGTAAACACTCAAAAG (Seq ID N. 18) | −3.093 | 110.5% | 0.996 |
| MS4A7 | CCCTCAAAGAGAGAAACCTGGA (Seq ID N. 19) | ATCAACAGGCAACACAGGATCT (Seq ID N. 20) | −3.162 | 107.1% | 0.964 |
| OSOX2 | CGTGTTCTCTCTGGAAACTGTTC (Seq ID N. 21) | GAACGTACCTCCTCATTGTCTGC (Seq ID N. 22) | −3.236 | 103.7% | 0.998 |
| PITRM1 | GGAAAATTCACACAGCAAGACA (Seq ID N. 23) | AGAGGCCGTACAAGAAGTGGT (Seq ID N. 24) | −3.192 | 105.7% | 0.997 |
| TGFb3 | AACTTCTGCTCAGGCCCTTG (Seq ID N. 25) | AGGCAGATGCTTCAGGGTTC (Seq ID N. 26) | −3.216 | 104.6% | 0.998 |
| PRC-1-201 | CCGTGTCTCGACTTCCTCCT (Seq ID N. 27) | CGTTGAGCTCCAGGTTCTCC (Seq ID N. 28) | −3.092 | 110.6% | 0.991 |
| GPR180 | GATTCTACGCCTGCATCCACT (Seq ID N. 29) | CCCTGCTAAGTTGTGGTGTGAA (Seq ID N. 30) | −3.076 | 111.4% | 0.996 |
| MMP9 | GCAAGCTGGACTCGGTCTT (Seq ID N. 31) | CCTGTGTACACCCACACCTG (Seq ID N. 32) | −2.198 | 185.1% | 0.953 |
| IGFBP6 | GAATCCAGGCACCTCTACCAC (Seq ID N. 33) | AGTCCAGATGTCTACGGCATGG (Seq ID N. 34) | −2.821 | 126.2% | 0.998 |
| IRS1 | CAGTTTCCAGAAGCAGCCAGAG (Seq ID N. 35) | GAGGATTTGCTGAGGTCATTTA (Seq ID N. 36) | −3.136 | 108.4% | 0.990 |
| IL6ST210 | CAGTGGTCACCTCACACTCCTC (Seq ID N. 37) | TTTGTCATTTGCTTCTATTTCCA (Seq ID N. 38) | −3.071 | 111.7% | 0.972 |
| IGF1 | TATCAGCCCCCATCTACCAAC (Seq ID N. 39) | TCTTGTTTCCTGCACTCCCTCT (Seq ID N. 40) | −3.012 | 102.3% | 0.998 |
| TNSF | TCCTCAGAGAGTAGCAGCTCACA (Seq ID N. 41) | CCTTGATGATTCCCAGGAGTT (Seq ID N. 42) | −2.628 | 140.2% | 0.759 |
| SERF1A | CCAGGAAATTAGCAAGGGAAAG (Seq ID N. 43) | CTTGTCTGCATAGACTTCTTCTCA (Seq ID N. 44) | −2.927 | 119.6% | 0.974 |

## REFERENCES CITED IN THE DESCRIPTION

**Non-patent literature cited in the description**

- **VAN DE VIJVER ; VAN'T VEER J.L. et al.** 70 gene good versus poor out come. *A gene-expression signature as a predictor of survival in breast cancer,* 2002, http://www.ncbi.nlm.nih.gov/pubmed/12490681
- **PAIK et al.** *recurrence-score,* 2004, http://www-ncbi.nlm.nih.gov/pubmed/15591335
- **MA XJ, WANG et al.** *two-gcnc-ratio model,* 2004, http://www.ncbi.nlm.nih.gov/pubmed/15193263
- **KOSNTANTOPOULOS NICKY.** *Insuline resistence,* 2011, https://www.researchgate.net/publication/47809153_A_gene_expression_signature_for_insuli n resistance